# EUROPEAN PATENT SPECIFICATION

(11) **EP 2 241 277 B1**
(45) Date of publication and mention of the grant of the patent: **20.06.2012**
(21) Application number: 10250762.1
(22) Date of filing: 13.04.2010
(51) Int. Cl.: A61B 17/34

(54) **Vibrating seal for a surgical trocar**
Vibrierende Dichtung für einen chirurgischen Trokar
Joint vibrant pour trocart chirurgical

(30) Priority: 14.04.2009 US 168962 P; 31.03.2010 US 751017
(43) Date of publication of application: 20.10.2010
(73) Proprietor: Tyco Healthcare Group LP, North Haven, CT 06473 (US)
(72) Inventor: Fischvogt, Gregory, Hamden, CT 06514 (US); Rockrohr, Brian, Westbury, CT 06705 (US)
(74) Representative: Soames, Candida Jane

(56) References cited:
- EP-A1- 2 116 201
- EP-A2- 1 997 444
- DE-C1- 3 238 832
- US-A- 5 584 850
- US-A1- 2006 041 245

## Description

### BACKGROUND

### 1. Field of the Disclosure

The present disclosure relates to surgical devices and more particularly to a seal assembly for use with a surgical access device during a minimally invasive surgical procedure.

### 2. Description of the Related Art

Minimally invasive surgical procedures including both endoscopic and laparoscopic procedures permit surgery to be performed on organs, tissues and .vessels far removed from an opening within the tissue. Laparoscopic and endoscopic procedures generally require that any instrumentation inserted into the body be sealed, i.e. provisions must be made to ensure that gases do not enter or exit the body through the incision as, for example, in surgical procedures in which the surgical region is insufflated. These procedures, typically employ surgical instruments which are introduced into the body through a cannula. The cannula has a seal assembly associated therewith. The seal assembly provides a substantially fluid tight seal about the instrument to preserve the integrity of the established pneumoperitoneum.

Minimally invasive procedures have several advantages over traditional open surgery, including less patient trauma, reduced recovery time, reduced potential for infection, etc. However, despite its recent success and overall acceptance as a preferred surgical technique, minimally invasive surgery, such as laparoscopy.. has several disadvantages. In particular, the maintenance of the seal about the surgical instrument has proved to be difficult in certain procedures, e.g., in procedures requiring extensive manipulation of the long narrow endoscopic instruments within a remote site. In addition, the force needed to insert the surgical instrument requires great effort by the user to overcome the seal's retention force.

EP 1997444 discloses a surgical access apparatus according to the pre characterizing portion of claim 1.

EP 2116201, which forms part of the state of the art according to Article 54 (3) EPC, discloses a trocar having a transducer for vibrating the trocar to remove fluids from the trocar.

### SUMMARY

The present invention, is directed to a surgical access apparatus for use during a surgical procedure. The surgical access apparatus allows instruments to pass through tissue to an underlying surgical area. The surgical access apparatus includes a housing mounted to an access member the housing having a seal member and a vibration source. The seal member is dimensioned to form a substantial sealing relation with a surgical instrument inserted therethrough and substantially close in the absence of the surgical instrument. The vibration source is connected to and causes vibration of the seal member.

The housing has a passageway therethrough and defines a longitudinal axis. The passageway is dimensioned to allow a surgical instrument to enter the housing and pass through the surgical access apparatus.

The vibration source can be a rotating eccentric mass, a mass moving in a linear path, an excited piezoelectric material, a crystal, an electro active polymer capable of expanding, or an electro active polymer capable of contracting. The crystal may be made of quartz.

The seal member has a proximal surface and a distal surface. Both the proximal and distal surfaces may be partially coated with a lubricant. Further, the seal member has an opening that forms the sealing relation with the surgical instrument. The opening may have a non-continuous cross-section. The non-continuous cross-section may be stepped shape.

The surgical access apparatus may have a second seal in the internal passage of the access member. The second seal may be a duckbill seal.

### BRIEF DESCRIPTION OF THE DRAWING(S)

Preferred embodiments of the present invention will be better appreciated by reference to the drawings wherein:
FIGS. 1 - 2 are perspective views of a cannula assembly and a seal assembly in accordance with the principles of the present invention;
FIG. 3 is a perspective view with parts separated of the cannula and seal assemblies of FIG. 1;
FIG. 4 is a side cross-sectional view of the cannula and seal assemblies;
FIG. 5 is a perspective view illustrating a seal assembly incorporated within the cannula housing;
FIG. 6 is a side cross-sectional view of an alternate embodiment of the seal assembly of FIG. 1;
FIG. 7 is a side cross-sectional view of an alternate embodiment of the seal assembly of FIG. 1; and
FIG. 8 is a side cross-sectional view of an alternate embodiment of the seal assembly of FIG. 1.

### DETAILED DESCRIPTION OF THE PREFERRED EMBODIMENT(S)

The surgical access apparatus of the present invention provides a substantial seal between a body cavity of a patient and the outside atmosphere before, during, and after insertion of a surgical instrument. Moreover, the surgical access apparatus of the present invention is capable of accommodating surgical objects of varying diameters, e.g., instruments from about 4.5 mm to about 15 mm, by providing a gas tight seal with each instrument when inserted. The flexibility of the present surgical access apparatus greatly facilitates endoscopic surgery where a variety of instruments having differing diameters are often needed during a single surgical procedure. The surgical access apparatus is further adapted to substantially close in the absence of a surgical instrument to maintain the integrity of the insufflated peritoneal cavity. In the following description, as is traditional the term "proximal" refers to the portion of the instrument closest to the operator while the term "distal" refers to the portion of the instrument remote from the operator.

The seal member may be readily incorporated into an access device, such as a conventional trocar device or cannula housing to provide the device with zero-closure and/or sealing around an instrument or other object. The surgical access apparatus contemplates the introduction and manipulation of various types of instrumentation adapted for insertion through a trocar and/or cannula assembly while maintaining a fluid tight interface about the instrumentation to preserve the atmospheric integrity of a surgical procedure from gas and/or fluid leakage. Specifically, the surgical access apparatus desirably minimizes the entry and exit forces required to insert and remove instrumentation into and from the body cavity. Examples of instrumentation include clip appliers, graspers, dissectors, retractors, staplers, laser probes, photographic devices, endoscopes and laparoscopes, tubes, and the like. Such instruments will be collectively referred to herein as "instruments or instrumentation".

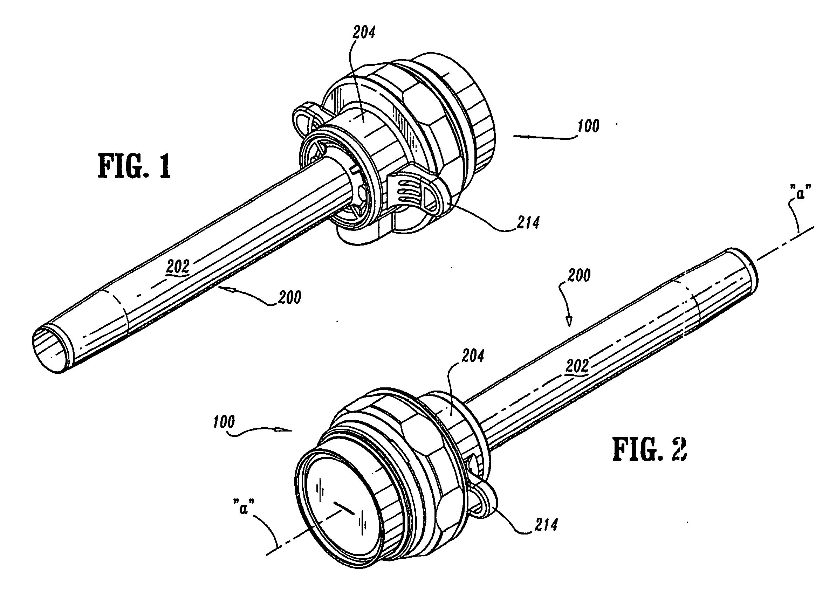

Referring now to the drawings, in which like reference numerals identify identical or substantially similar parts throughout the several views, FIGS. 1-2 illustrate a seal assembly 100 of the present invention mounted to an access member 200, such as a cannula or trocar assembly. The cannula assembly may be any conventional cannula suitable for the intended purpose of accessing a body cavity and typically defines a passageway permitting introduction of instruments therethrough. Access member 200 is particularly adapted for use in laparoscopic surgery where the peritoneal cavity is insufflated with a suitable gas, e.g., CO₂, to raise the cavity wall from the internal organs therein. Access member 200 is typically used with an obturator assembly (not shown) which may be blunt, a non-bladed, or a sharp pointed instrument positionable within the passageway of the access member 200. The obturator assembly is utilized to penetrate the abdominal waii or introduce the access member 200 through the abdominal wall, and then subsequently is removed from the cannula assembly to permit introduction of the surgical instrumentation utilized to perform the procedure through the passageway.

Access member 200 includes cannula sleeve 202 and cannula housing 204. Cannula sleeve 202 defines a longitudinal axis "a" extending along the length of sleeve 202. Sleeve 202 further defines an internal longitudinal passage 118 dimensioned to permit passage of surgical instrumentation. Sleeve 202 may be formed of stainless steel or other rigid materials such as a polymeric material or the like. Based on the material selected, sleeve 202 may be clear or opaque. The diameter of sleeve 202 may vary, but typically ranges from about 4.5 to about 15 mm for use with the seal assembly 100 of the present invention.

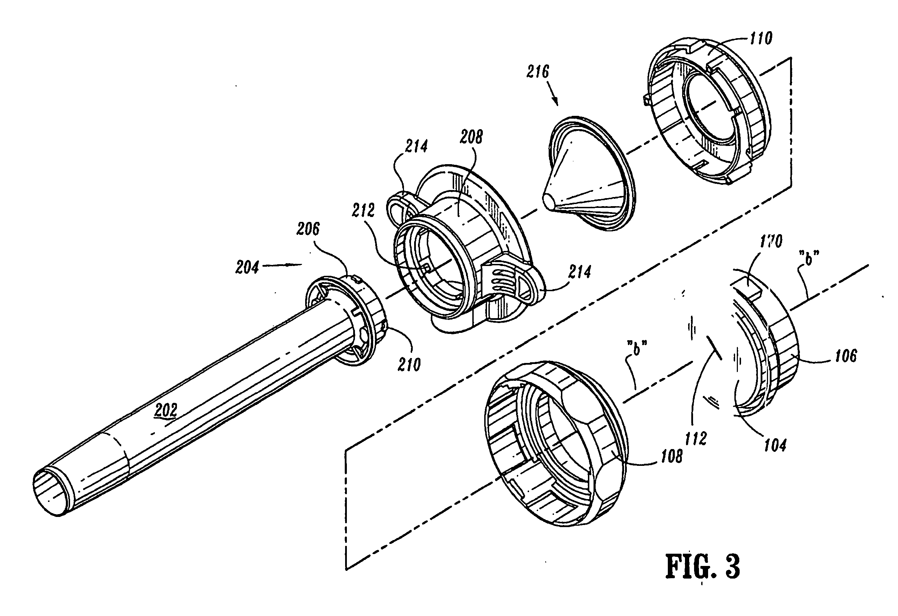

As shown in FIGS. 3-4, cannula housing 204 includes two components, specifically, housing flange 206 is attached to the proximal end of cannula sleeve 202 and main housing 208. Main housing 208 is connectable to housing flange 206 through a bayonet coupling consisting of radially spaced tongues 210 on the exterior of housing flange 206 and corresponding recesses 212 within the interior of main housing 208. Tongues 210 are receivable within recesses 212. Thereafter, housing flange 206 and main housing 208 are rotated to securely lock the tongues 210 within the recesses 212. Other conventional means, e.g., a threaded snap fit, ultrasonic welding or any other means envisioned by one skilled in the art including, e.g., adhesive means, may be incorporated to connect housing flange 206 and main housing 208.

Main housing 208 further includes diametrically opposed housing grips 214 dimensioned and arranged for gripping engagement by the fingers of the user. Additionally or alternatively, suture anchors may extend from main housing 208. Although shown and described as two components, cannula housing 204 may be a single component and attached to cannula sleeve 202 by any of the aforementioned means. The housing flange 206, or the housing flange 206 and main housing 208, may be integrally formed with cannula sleeve 202.

Referring now to FIGS. 3 - 4, in conjunction with FIGS. 1-2, seal assembly 100 will be discussed in detail. Seal assembly 100 includes a housing, generally identified as reference numeral 102, and a seal member 104. Housing 102 contains the seal member 104 and a vibration source 150, and defines the outer valve or seal body of the seal assembly 100.

Housing 102 defines central housing axis "b" which is preferably parallel to the axis "a" of cannula sleeve 202 and, more specifically, coincident with the longitudinal axis "a". Housing 102 incorporates three housing components, namely, proximal, distal and inner housing components 106, 108, 110, respectively. The housing 102 is formed with an internal passageway 114. The passageway 114 allows surgical instruments access to the surgical site. Assembly of housing components 106, 108, 110 may be done by any of the aforementioned connection means discussed with respect to cannula housing 204. Although shown and described as three components, it is appreciated that housing 102 may be a single component having the seal member 104 mounted therein.

With particular reference to FIGS. 3-4, seal member 104 will be discussed in detail. The seal member 104 is mounted to proximal housing component 106 through, e.g., conventional means, such as by adhering the seal member 104 to the component 106 or molding the seal member 104 in the component 106. Seal member 104 is fabricated from an elastomer such as a soft urethane, silicone, etc. and has compressible characteristics to permit the seal to receive objects having a variety of sizes, to conform and form a seal about the outer surface of the inserted object, and to close upon removal of the object.

Seal member 104 includes a single slit 112 advantageously dimensioned to permit reception and passage of a surgical instrument. In particular, slit 112 opens to permit passage of the surgical instrument whereby the internal portions defining the slit 112 engage the instrument in sealed relation therewith. The slit 112 is further adapted to assume a substantially closed position upon removal of the instrument. In this position, seal member 104 prevents the egress of gaseous matter through seal housing 102. Slit 112 may have shapes other than that of a single linear slit, such as "t"-shaped, "x" shaped, helical, etc. Still further, slit 112 may have a non-constant cross section. The cross-section of slit 112 may have a stepped shape 122.

Seal member 104 may be frusto-conical in shape and define an aperture for sealed reception of the instrument. Seal member 104 is a flat disc-shaped valve. The seal member 104 may comprise a flat disc-shaped member including a fabric material molded with an elastomer.

A preferred material is a synthetic material such as nylon, Kevlar (Trademark of E.I. DuPont de Nemours and Company) or any other material that will expand and compress about an instrument inserted therethrough. The fabric may have a coating 116 of urethane, silicon or other flexible lubricious materials to facilitate passage of an instrument or other object through the seal member 104. The fabric is desirably constructed of a material and/or arranged so that the fabric forms a constriction or closure.

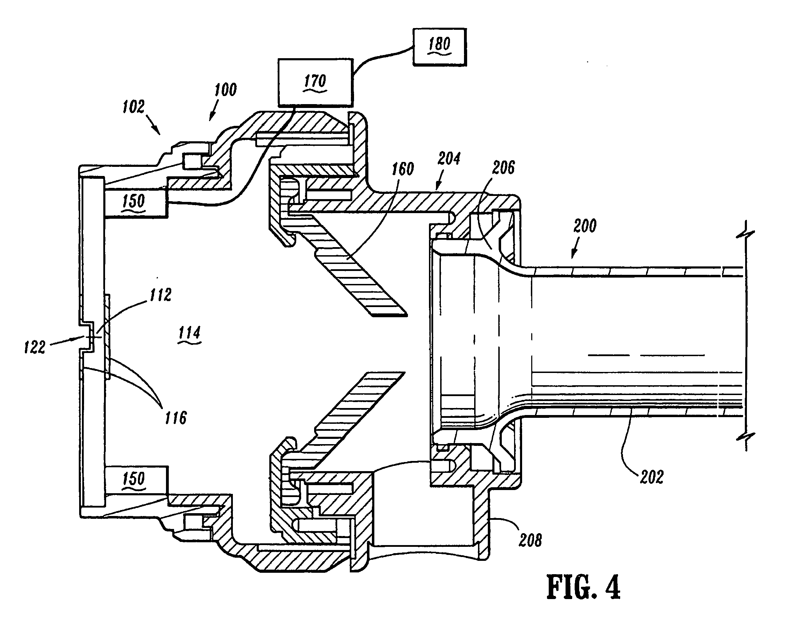

The seal member 104 is connected to a vibration source 150. The connection between the vibration source 150 and the seal member 104 causes any movement or vibration, e.g., oscillation, of the vibration source 150 to be translated to the seal member 104, thereby causing the seal member 104 to vibrate, also. The vibration source 150 may be a rotating eccentric mass, a mass moving in a linear path, an excited piezoelectric material, a crystal (i.e. quartz), an electro active polymer capable of expanding, or an electro active polymer capable of contracting. The vibration source 150 activates when it is supplied with electrical power 180 illustrated schematically in FIG.4. The electric power may be a battery, AC source, DC source, capacitor, or any other power source appreciated by one skilled in the art. The power source 180 may be external of housing 102, mounted to the housing 102, or incorporated within the housing 102. The electrical power may be supplied by the manual engagement of an electrical switch 170 schematically illustrated in FIG. 3. Electric switch 170 may be mounted directly to housing 102 or external of the housing 102. Alternatively, an object coming into contact with or near a sensor (not shown), within the housing, may act as an electrical switch 170 and supply electrical power 180 to the vibration source 150.

With continued reference to FIG. 3, in conjunction with FIGS. 1-2, housing 102 further includes a second seal 160. The second seal may be a duckbill seal.

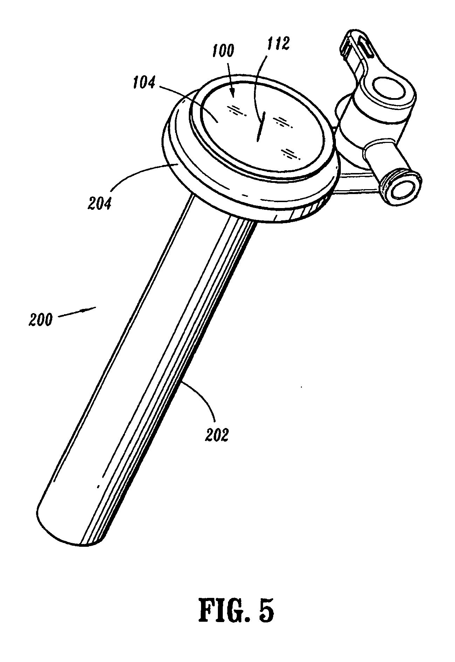

Seal assembly 100 may be associated with, or joined to, access member 200 in a variety of ways. In a preferred embodiment, housing 102 of seal assembly 100 and cannula housing 204 of access device 200 are adapted to detachably engage each other, e.g., through a bayonet lock, threaded attachment, latching attachment, or like mechanical means. In further embodiments, cannula housing 208 and seal member 160 may be omitted and seal assembly 100 may be removably or permanently attached to flange 206. The seal assembly may be mounted to cannula assembly 200 before during or after application of the cannula assembly within the operative site. Alternatively, the seal assembly 100 may be built within cannula housing 204 as depicted in FIG. 5.

The use of the surgical access apparatus 100 and access member 200 in connection with introduction of a surgical instrument will be discussed. Seal assembly 100 is mounted to access member 200, and access member 200 is introduced into an insufflated abdominal cavity. A user engages a manual switch (not shown) causing both the vibration source 150 and the seal member 104 to vibrate. An object, e.g., an instrument, is inserted into surgical access apparatus 100 through the vibrating slit 112 whereby the portions defining the slit 112 stretch to accommodate the instrument diameter, as necessary.

The instrument is distally passed through the vibrating seal member 104 in sealed relation therewith and into the body cavity to perform the desired procedure. The force required to pass the instrument through vibrating slit 112 is less than the force required to insert the instrument through a static seal member. The reduction of required force is a result of the difference between static and kinetic friction. The coefficient of static friction is usually higher than the coefficient of kinetic friction. Other instruments may be introduced through the seal assembly 100 and cannula assembly to perform further operative techniques as desired. The seal member 104 continues to vibrate while the instrument is within the slit 112. In the alternative, the power source 180 is disengaged from the vibration source 150 while the instrument is within the slit 112. Once the instrument is removed, the slit 112 of the seal member 104 substantially closes to a zero-closure position to maintain the integrity of the established pneumoperitoneum.

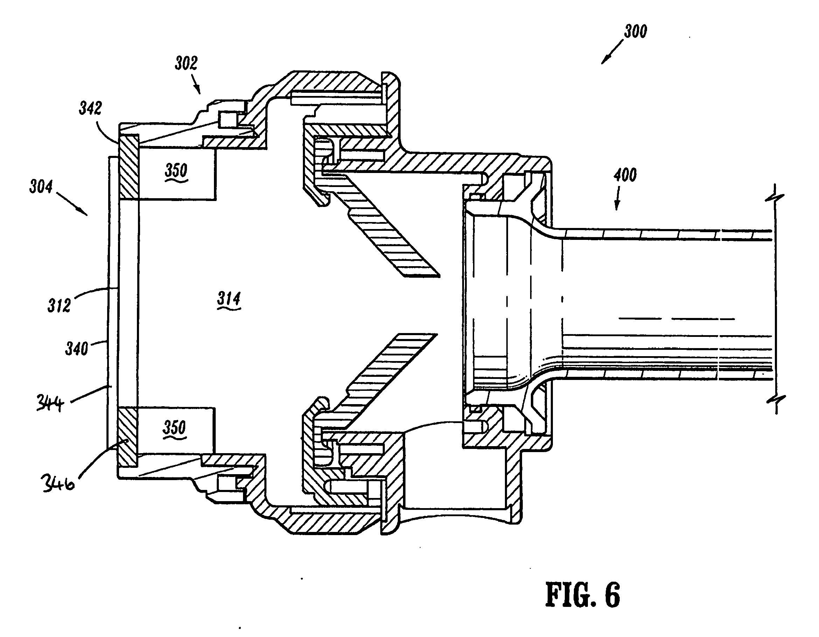

In another embodiment, shown as FIG. 6, surgical access apparatus 300 includes a housing 302, an elongate member 400, a seal unit 304, and a vibration source 350. The housing 302 and the elongate member 400 are mounted together to define a longitudinal axis "a" and an internal passage 314 from a proximal end, of the housing 302, to a distal end, of the elongate member 400. The passage 314 is sized to permit the passage of a surgical instrument therethough. The seal unit 304 is mounted across the passage 314 and includes a first seal component 340 and a second seal component 342.

The first seal component 340 has an access portion 312 dimensioned to form a substantial sealing relation with the surgical instrument when inserted, and to substantially close in the absence of the surgical instrument. The first seal component 340 is made from a first material 344 that is generally softer and more pliable than a second material 346 forming second seal component 342. The second seal material 346 may include a relatively hard material, e.g., a metal, a plastic, or any of the aforementioned seal materials specifically treated to increase its respective rigidity. The second seal component 342 is more rigid than the first seal component 340. The first material 344 of the first seal component 340 is mounted to, or embedded in, the second material 346 of the second seal component 342.

The first seal component 340 is mounted to the second seal component 342 through conventional means including adhesives, cements, etc. Alternatively, the first seal component 340 may be cast molded with the second seal component 342 during manufacture and secured to the second seal component 342 during curing of the first seal component 340. The second seal component 342 defines an opening or access portion 312 dimensioned to permit passage of an object. The second seal component 342 provides a rigid band around the outside of the first seal component. The rigid band more efficiently transmits vibration from the vibration source 350 into the first seal member 340.

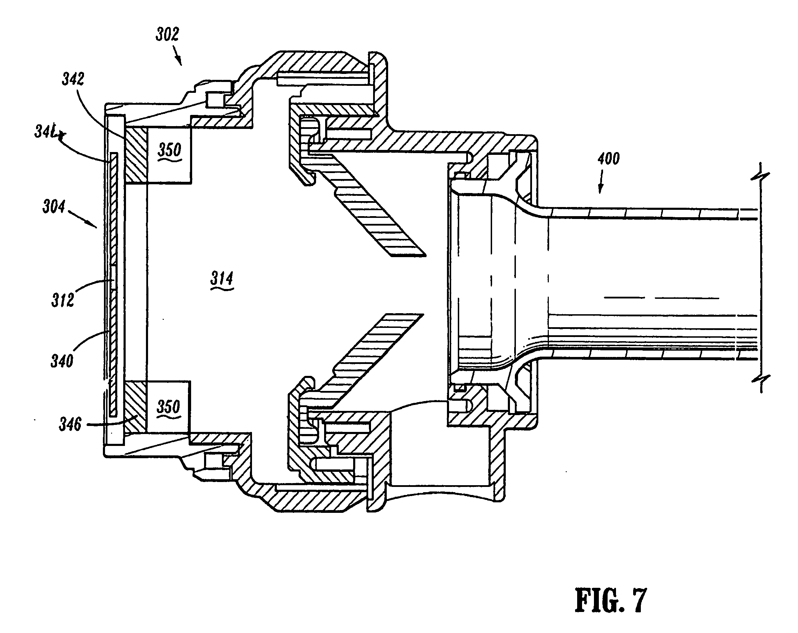

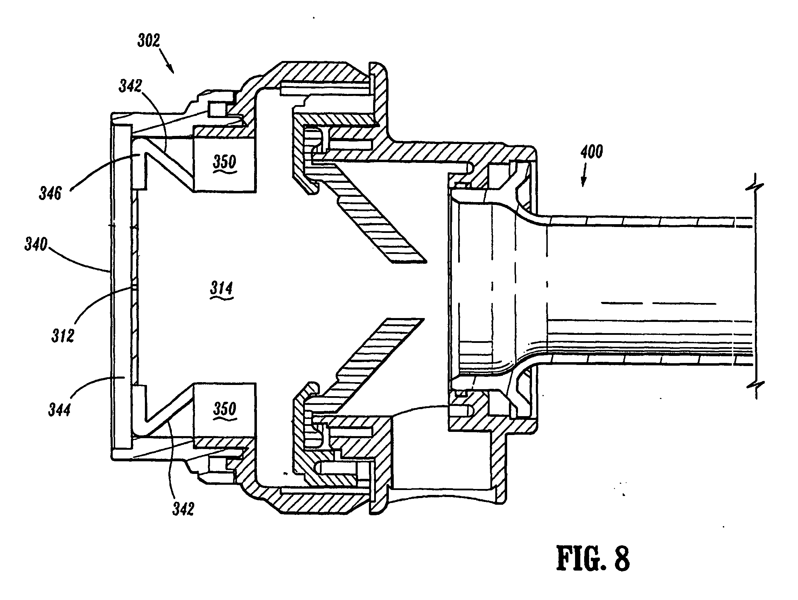

FIG. 7 and 8 illustrate various alternate embodiments of the surgical access apparatus 300 of FIG. 6. Each embodiment includes a seal unit 304 fabricated from a first, generally soft, material 344 which is mounted to a second, generally rigid material 346. The combination of materials aids in providing movement at the slit 312 during vibration of the vibration source 350. In one embodiment, the first material may be any suitable material identified hereinabove in connection with the embodiment of FIGS. 1-4, including, for example urethane. The first material may comprise any elastomeric material. Additionally or alternatively, the first material is a fabric material including a woven, braided or knitted material of the type discussed hereinabove in connection with the embodiment of FIGS. 1-4.

While the invention has been particularly shown, and described with reference to the preferred embodiments, it will be understood by those skilled in the art that various modifications and changes in form and detail may be made therein without departing from the scope of the claims.

## Claims

1. A surgical access apparatus for passing through tissue to an underlying surgical area, which comprises:
an access member (200) having an internal passage (118);
a housing (102) defining a longitudinal axis and having a passageway (114) therethrough, the passageway (114) being dimensioned to permit passage of the surgical instrument, wherein the housing (204) is mounted to the access member (200); and
a seal member (104) disposed in the passageway (114), wherein the seal member (104) is dimensioned to form a substantial sealing relation with a surgical instrument inserted therethrough and substantially closed in the absence of the surgical instrument; **characterized in that**
the seal member (104) comprises a flat disc shaped valve having a slit (112) therein to permit passage of the surgical instrument;
and **in that** the apparatus further comprises a vibration source (150) operatively connected to the seal member (104) for effecting vibrations of the seal member (104).

2. The surgical access apparatus according to claim 1, wherein the vibration source (150) is selected from the group consisting of a rotating eccentric mass, a mass moving in a linear path, an excited piezoelectric material, a crystal, an electro active polymer capable of expanding, and an electro active polymer capable of contracting.

3. The surgical access apparatus according to claim 2, wherein the crystal is quartz.

4. The surgical access apparatus according to any preceding claim, wherein the seal member (104) comprises a lubricious coating.

5. The surgical access apparatus according to any preceding claim, wherein the seal member (104) has a proximal surface defining an opening, the opening having a stepped shape.

6. The surgical access apparatus according to any preceding claim, further comprising a second seal (160) disposed in the internal passage (114).

7. The surgical access apparatus according to claim 6, wherein the second seal (160) is a duckbill seal.

## Patentansprüche

1. Chirurgische Zugriffsvorrichtung, um durch Gewebe in einen darunter liegenden chirurgischen Bereich zu führen, die Folgendes umfasst:
ein Zugriffselement (200) mit einem internen Durchgang (118);
ein Gehäuse (102), das eine Längsachse definiert und einen Verbindungsweg (114) **dadurch** aufweist, wobei der Verbindungsweg (114) abgemessen ist, um den Durchgang des chirurgischen Instruments zu ermöglichen, wobei das Gehäuse (204) auf das Zugriffselement (200) montiert ist; und
ein Dichtungselement (104), das im Verbindungsweg (114) angebracht ist, wobei das Dichtungselement (104) abgemessen ist, um eine wesentliche Dichtungsbeziehung mit einem chirurgischen Instrument zu bilden, das **dadurch** eingeführt ist und bei Abwesenheit des chirurgischen Instruments im Wesentlichen geschlossen ist, **dadurch gekennzeichnet, dass**:
das Dichtungselement (104) ein flaches scheibenförmiges Ventil mit einem Schlitz (112) darin umfasst, um den Durchgang des chirurgischen Instruments zu ermöglichen;
und **dadurch**, dass die Vorrichtung weiter eine Vibrationsquelle (150) umfasst, die in Betrieb mit dem Dichtungselement (104) verbunden ist, um Vibrationen des Dichtungselements (104) durchzuführen.

2. Chirurgische Zugriffsvorrichtung nach Anspruch 1, wobei die Vibrationsquelle (150) ausgewählt ist aus der Gruppe bestehend aus einer exzentrischen Drehmasse, einer Masse, die sich auf einem linearen Pfad bewegt, einem erregten piezoelektrischen Material, einem Kristall, einem elektroaktiven Polymer, das dazu in der Lage ist, sich auszudehnen, und einem elektroaktiven Polymer, das dazu in der Lage ist, zu schrumpfen.

3. Chirurgische Zugriffsvorrichtung nach Anspruch 2, wobei das Kristall ein Quarz ist.

4. Chirurgische Zugriffsvorrichtung nach einem der vorhergehenden Ansprüche, wobei das Dichtungselement (104) eine schmierende Beschichtung umfasst.

5. Chirurgische Zugriffsvorrichtung nach einem der vorhergehenden Ansprüche, wobei das Dichtungselement (104) eine proximale Fläche aufweist, die eine Öffnung definiert, wobei die Öffnung eine gestufte Form aufweist.

6. Chirurgische Zugriffsvorrichtung nach einem der vorhergehenden Ansprüche, weiter umfassend eine zweite Dichtung (160), die im inneren Durchgang (114) angeordnet ist.

7. Chirurgische Zugriffsvorrichtung nach Anspruch 6, wobei die zweite Dichtung (160) eine Entenschnabeldichtung ist.

## Revendications

1. Appareil d'accès chirurgical pour passer à travers le tissu jusqu'à une zone chirurgicale sous-jacente, qui comprend :
un élément d'accès (200) ayant un passage interne (118) ;
un logement (102) définissant un axe longitudinal et ayant un passage (114) à travers lui, le passage (114) étant dimensionné de façon à permettre le passage de l'instrument chirurgical, dans lequel le logement (204) est monté sur l'élément d'accès (200) ; et
un élément de joint (104) disposé dans le passage (114), dans lequel l'élément de joint (104) est dimensionné de façon à former une relation d'étanchéité sensible avec un instrument chirurgical inséré à travers lui et sensiblement fermé en l'absence de l'instrument chirurgical , **caractérisé en ce que**
l'élément de joint (104) comprend une vanne en forme de disque plat ayant une fente (112) à l'intérieur, pour permettre le passage de l'instrument chirurgical ;
et **en ce que** l'appareil comprend en outre une source de vibration (150) opérationnellement raccordée à l'élément de joint (104) pour faire vibrer l'élément de joint (104).

2. Appareil d'accès chirurgical selon la revendication 1, dans lequel la source de vibration (150) est choisie dans le groupe constitué d'une masse excentrique rotative, d'une masse se déplaçant sur un chemin linéaire, d'un matériau piézoélectrique excité, d'un cristal, d'un polymère électro-actif capable de se dilater et d'un polymère électro-actif capable de se contracter.

3. Appareil d'accès chirurgical selon la revendication 2, dans lequel le cristal est un quartz.

4. Appareil d'accès chirurgical selon l'une quelconque des revendications précédentes, dans lequel l'élément de joint (104) comprend un revêtement lubrifié.

5. Appareil d'accès chirurgical selon l'une quelconque des revendications précédentes, dans lequel l'élément de joint (104) a une surface proximale définissant une ouverture, l'ouverture ayant une forme étagée.

6. Appareil d'accès chirurgical selon l'une quelconque des revendications précédentes, comprenant en outre un second joint (160) disposé dans le passage interne (114).

7. Appareil d'accès chirurgical selon la revendication 6, dans lequel le second joint (160) est un joint en bec de canard.
